# EUROPEAN PATENT APPLICATION

(11) **EP 1 741 440 A1**
(43) Date of publication of application: **10.01.2007**
(21) Application number: 05106245.3
(22) Date of filing: 08.07.2005
(51) Int. Cl.: A61K 38/17, A61P 9/00, A61P 3/04, A61P 3/10, A61P 3/06

(54) **Use of BPI protein for the treatment of disorders of the metabolism and cardiovascular disorders**

(71) Applicant: Mellitus S.L., 17003 Girona (ES)
(72) Inventor: Fernández- Real, José-Manuel, 17003, Girona (ES); Ricart Engel, Wilfredo, 17003 Girona (ES)
(74) Representative: Barlocci, Anna

(57) **Abstract**

Use of a bactericidal/permeability-increasing (BPI) protein product for the manufacture of a medicament for therapeutic or preventive treatment of a disease involving a clinical disorder associated with insulin resistance in a human.

## Description

### FIELD OF THE INVENTION

The present invention relates to the use of a bactericidal/permeability-increasing (BPI) protein product for the manufacture of a medicament for therapeutic or preventive treatment of a disease involving a clinical disorder associated with insulin resistance in a human.

### BACKGROUND OF THE INVENTION

The outer membrane of Gram-negative bacteria contains the complex glycolipid lipopolysaccharide (LPS). When present in the mammalian bloodstream, LPS elicits a powerful immune response that may include fever, hypotension, multiple organ failure and, in severe cases, septic shock and death. In mammals, two related LPS-binding proteins have been characterized that mediate the biological effects of LPS and participate in the innate immune response to bacterial infection: LPS-binding protein (LBP) and bactericidal/permeability-increasing protein (BPI). LBP is a plasma protein that enhances the inflammatory response to LPS, whereas BPI is found in lysosomal granules of polymorphonuclear neutrophils (PMN), is bactericidal, and neutralizes the toxic effects of LPS.

Human BPI is a protein of 456 residues and was originally reported to be a potent bactericidal agent active and highly specific against a broad range of Gram-negative bacterial species whereas non-toxic for other microorganisms and eukaryotic cells (US patents 5198541, 5641874, 5948408, 5980897 and 5523288). BPI has later demonstrated to be also bactericidal for Gram-positive bacteria (WO 95/19180), antifungal (WO 95/19179), anti-protozoan (WO 96/01647), anti-chlamydial (WO 98/06415) and anti-mycobacterial (WO 94/20129).

Other documents in the art describe the use of BPI or variants/fragments of BPI for treatment of meningococcemia in humans, hemorrhage due to trauma in humans, burn injury, ischemia/reperfusion injury and depressed RES/liver resection. See e.g. WO 97/42966, WO 97/44056, WO 96/30037, US 5578568, WO 95/10297. BPI protein products also neutralize the anticoagulant activity of exogenous heparin (US 5348942), neutralize heparin *in vitro* (US 5854214) and are useful for treating chronic inflammatory diseases states such as rheumatoid and reactive arthritis, for inhibiting endothelial cell proliferation, and for inhibiting angiogenesis and for treating angiogenesis-associated disorders including malignant tumors, ocular retinopathy and endometriosis (WO 94/20128).

Document WO 02/055099 relates generally to methods of using a BPI protein product to enhance perivascular cells ("pericytes") proliferation in conditions where pericyte proliferation is desirable, such as complications of diabetes (e.g. diabetic retinopathy or diabetic polyneuropathy). It also describes methods of inhibiting pericyte proliferation, desirable in conditions where pericyte proliferation is deleterious. Such conditions include hypertension, vascular disease and atherosclerosis, among others. The inhibition of pericyte proliferation is achieved by reducing the effect of BPI, i.e., using inhibitors capable of inhibiting the type of proliferation induced by BPI protein product.

### SUMMARY OF THE INVENTION

The problem to be solved by the present invention is to provide an alternative new medical use for BPI.

The solution is based on detailed work (see working examples herein) wherein the present inventors identified that BPI protein can improve insulin resistance of the subjects having a disease involving a clinical disorder associated with insulin resistance. When insulin resistance is improved, endogenous insulin action is enhanced and then, blood glucose levels lower to a normal level.

Such a basic understanding of a glucose "normalizing" effect of BPI opens the door to use it to treat hyperglycemia in a number of relevant diseases involving a clinical disorder associated with insulin resistance.

A relevant disease may for instance be type 2 diabetes mellitus. Use of BPI to lower the level of glucose in blood and thereby get a normalization of the level of glucose, in a human with type 2 diabetes is clinically clearly different to the use of BPI for vascular treatment of diabetes such as diabetic retinopathy as described in WO 02/055099.

In this context, it is relevant to mention that WO 02/055099 describes that BPI inhibitors shall be used for treatment of diseases such as hypertension and atherosclerosis. As explained herein, based on the insulin resistance improving effect of BPI, the present inventors have, contrary to what is disclosed in WO 02/055099, identified that BPI may be useful for treatment of diseases such as hypertension and atherosclerosis.

Accordingly, a first aspect of the invention relates to the use of a bactericidal/permeability-increasing (BPI) protein product for the manufacture of a medicament for therapeutic or preventive treatment of a disease involving a clinical disorder associated with insulin resistance in a mammal (preferably a human), in order to lower the level of glucose in blood and thereby get a normalization of the level of glucose, wherein the disease is a disease selected from the group consisting of: type 2 diabetes mellitus, obesity, glucose intolerance, metabolic syndrome, arterial hypertension, cardiovascular disease, dyslipidemia, and functional ovaric hyperandrogenism.

This first aspect may alternatively be formulated as a method for therapeutic or preventive treatment of a disease involving a clinical disorder associated with insulin resistance in a mammalian (preferably a human), in order to to lower the level of glucose in blood and thereby get a normalization of the level of glucose, wherein the disease is a disease as described for the first aspect above, comprising administering to a mammal (preferably a human) in need thereof an effective amount of BPI protein product.

An advantage of using BPI protein is that it improves insulin resistance and thereby normalizes blood glucose levels. On the contrary, if insulin is given to e.g. type 2 diabetes patients, glucose levels go down in a more uncontrollable manner. Said in another way, one has to continuously and precisely control the amount of administrated insulin and continuously monitor the concentrations of glucose in order not to get too low glucose levels. Since BPI protein merely normalizes the glucose level, the risk of too low glucose levels is absent. This offers the possibility of e.g. administrating the BPI protein as a depot (e.g. an injectable depot) to the patient. The depot can be made with an adequate release profile of BPI protein and the patient can thereby, in a comfortable easy way, get a continued improved insulin resistance and thereby continued normalized blood glucose levels. See below for further details.

The term "normalization" in the sentence "in order to to lower the level of glucose in blood and thereby get a normalization of the level of glucose" of the first aspect of the invention, should be understood as a clinical relevant lowering of the level of glucose in blood. This is to get a level of glucose that is close to a normal level of glucose in a healthy person.

A second aspect of the invention relates to a kit for detecting a predisposition to a disease involving a clinical disorder associated with insulin resistance in a human, wherein the disease is a disease according to the first aspect, comprising appropriate means for detecting, in a separated sample from the human, the presence or the absence of an adenine in the polymorphism at 3' untranslated region of the human BPI protein gene, said polymorphism identified as rs1131847 in NCBI, Entrez SNP, and said polymorphism located at nucleotide 1524, 5'-3', of the complete BPI protein gene referenced as NCBI NM_001725, Entrez Nucleotide.

Embodiments of the present invention are described below, by way of examples only.

### DETAILED DESCRIPTION OF THE INVENTION

### Suitable BPI

Below are described examples of suitable BPI protein products.

The molecular weight of human BPI is approximately 55 kD. The amino acid sequence of the human BPI protein and the nucleic acid sequence of DNA encoding the protein are reported in NCBI GenBank Accession Number NM_001725. Human BPI protein has been isolated from PMNs by acid extraction combined with either ion exchange chromatography (Elsbach, J.Biol. Chem., 1979, vol. 254 pp. 11000) or *E. coli* affinity chromatography (Weiss, et al., Blood, 1987, vol. 69, pp. 652). BPI obtained in such a manner is referred to herein as natural BPI.

As used herein, the term "BPI protein product" includes naturally or recombinantly produced BPI protein; natural, synthetic, or recombinant biologically active polypeptide fragments of BPI protein; biologically active polypeptide variants of BPI protein or fragments thereof, including hybrid fusion proteins or dimers; biologically active polypeptide analogs of BPI protein or fragments or variants thereof, including cysteine-substituted analogs; or BPI-derived peptides.

BPI protein is from mammalian origin and preferably, from human origin.

The BPI protein products administered according to this invention may be generated and/or isolated by any means known in the art. US 5198541 and 5641874 disclose recombinant genes encoding, and methods for expression of, BPI proteins including recombinant BPI holoprotein, referred to as rBPI and recombinant fragments of BPI. WO 93/23540 discloses novel methods for the purification of recombinant BPI protein products expressed in and secreted from genetically transformed mammalian host cells in culture and discloses how one may produce large quantities of recombinant BPI products suitable for incorporation into stable, homogeneous pharmaceutical preparations.

Biologically active fragments of BPI (BPI fragments) include biologically active molecules that have the same or similar amino acid sequence as a natural human BPI holoprotein, except that the fragment molecule lacks N-terminal amino acids, internal amino acids, and/or C-terminal amino acids of the holoprotein, including those described in US 5198541 and US 5641874. Nonlimiting examples of such fragments include an N-terminal fragment of natural human BPI of approximately 25 kD, described in Ooi et al., J. Exp. Med., 1991, vol. 174, pp. 649, or the recombinant expression product of DNA encoding N-terminal amino acids from 1 to about 193 to 199 of natural human BPI, described in Gazzano-Santoro et al., Infect. Immun. 1992, vol. 60, pp. 4754-61, and referred to as BPI23. Another fragment consisting of residues 10-193 of BPI has been described in WO 99/66044. Other examples include dimeric forms of BPI fragments, as described in WO 95/24209.

Biologically active variants of BPI (BPI variants) include but are not limited to recombinant hybrid fusion proteins, comprising BPI holoprotein or biologically active fragment thereof and at least a portion of at least one other polypeptide, or dimeric forms of BPI variants. Examples of such hybrid fusion proteins and dimeric forms are described in WO 93/23434 and include hybrid fusion proteins comprising, at the N-terminal end, a BPI protein or a biologically active fragment thereof and, at the C-terminal end, at least one constant domain of an immunoglobulin heavy chain or allelic variant thereof (e.g., a BPI-Ig fusion protein).

Biologically active analogs of BPI (BPI analogs) include but are not limited to BPI protein products wherein one or more amino acid residues have been replaced by a different amino acid. For example, WO 94/18323 discloses polypeptide analogs of BPI and BPI fragments wherein a cysteine residue is replaced by a different amino acid. A stable BPI protein product described by this application is the expression product of DNA encoding from amino acid 1 to approximately 193 or 199 of the N-terminal amino acids of BPI holoprotein, but wherein the cysteine at residue number 132 is substituted with alanine and is designated as rBPI21. Similarly, an analog consisting of residues 10-193 of BPI in which the cysteine at position 132 is replaced with an alanine (designated "rBPI (10-193) C132A) has been described in WO 99/66044. Other examples include dimeric forms of BPI analogs; e. g. WO 95/24209.

Other BPI protein products useful according to the invention are peptides derived from or based on BPI produced by synthetic or recombinant means (BPI-derived peptides), such as those described in WO 97/04008.

Exemplary BPI protein products include recombinantly-produced N-terminal analogs or fragments of BPI, especially those having a molecular weight of approximately between 20 to 25 kD such as rBPI21, rBPI23, rBPI (10-193) C132A, dimeric forms of these N-terminal polypeptides (e. g., rBPI42 dimer), or BPI-derived peptides. Exemplary BPI-derived peptides include peptides derived from domain 11 of BPI, such as XMP.679.

In a preferred embodiment the BPI protein product is a protein that comprises the following amino acid sequence: V N P G V V V R I S Q K G L D Y A S Q Q G T A A L Q K E L K R I K I P D Y S D S F K I K H L G K G H Y S F Y S M D I R E F Q L P S S Q I S M V P N V G L K F S I S N A N I K I S G K W K A Q K R F L K M S G N F D L S I E G M S I S A D L K L G S N P T S G K P T I T A S S C S S H I N S V H V H I S K S K V G W L I Q L F H K K I E S A L R N K M N S Q V C E K V T N S V S S K L Q P Y F Q T L, referred to as BPI21 (SEQ ID NO:1), which is a recombinant expression product of DNA encoding N-terminal amino acids from 1 to about 193 of natural human BPI (Gazzano-Santoro et al., Infect. Immun. 1992, vol. 60, pp. 4754-61), but wherein the cysteine at residue number 132 of the natural human BPI has been substituted with alanine.

They are also preferred embodiments, fragments of this amino acid sequence, such as: fragment A S Q Q G T A A L Q K E L K R I K I P D Y S D S F K I K H (SEQ ID NO:2); fragment S S Q I S M V P N V G L K F S I S N A N I K I S G K W K A Q K R F L K (SEQ ID NO:3); and fragment V H V H I S K S K V G W L I Q L F H K K I E S A L R N K (SEQ ID NO:4).

### Disease involving a clinical disorder associated with insulin resistance

Without being limited to theory, clinical symptoms of the diseases of the first aspect of the invention are believed to be associated with some kind of a chronic inflammation condition.

According to the art acute condition may be defined as condition that is severe and sudden in onset. Symptoms appear, change, or worsen rapidly. A chronic condition, by contrast, is continuous or persistent/maintained over an extended period of time. A chronic condition is one that is long-standing, not easily or quickly resolved.

Accordingly, in a preferred embodiment the disease of the first aspect is a disease which is involving a chronic inflammation condition. This chronic inflammation condition is characterized by an elevated high-sensitivity C-reactive protein in blood, and particularly by a level of C-reactive protein in blood higher than 2 mg/l.

Clinical symptoms of all the mentioned diseases, of the first aspect, are in one way or the other known to be associated with some kind of insulin resistance.

The term "Insulin resistance" shall herein be understood according to the art. Insulin resistance occurs when muscle, fat, and liver cells do not use insulin properly. The pancreas tries to keep up with the demand for insulin by producing more. Eventually, the pancreas cannot keep up with the body's need for insulin, and excess glucose builds up in the bloodstream ("hyperglycemia").

Many people with insulin resistance have high levels of blood glucose and high levels of insulin circulating in their blood at the same time. Blood glucose levels stay elevated and the body responds by producing more insulin, provoking yet greater intolerance in response to the elevated insulin levels that are then present in the blood. People with blood glucose levels that are higher than normal but not yet in the diabetic range have "pre-diabetes". Doctors sometimes call this condition "impaired fasting glucose" (IFG) or "impaired glucose tolerance" (IGT), depending on the test used to diagnose it. Type 2 diabetes results when the pancreas can no longer maintain this level of insulin production.

It is within the general knowledge of the skilled person to identify if e.g. a human has one of the diseases of the first aspect. Reference is e.g. made to the National Library of Medicine, USA (NLM) website: (http://www.nlm.nih.gov), where details of these diseases are described. Below relevant clinical details are discussed.

"Glucose intolerance" is a condition closely associated with type 2 diabetes. As explained above doctors sometimes call this condition "impaired fasting glucose" (IFG) or "impaired glucose tolerance" (IGT), depending on the test used to diagnose it. High blood sugar levels are present, but not high enough to warrant a diagnosis of diabetes. Insulin is still produced by the body, but in insufficient amounts to control blood sugar levels effectively. Glucose intolerance is often considered an early warning sign for type 2 diabetes: one to ten percent of people with glucose intolerance develop type 2 diabetes every year. The causes of glucose intolerance are similar to those identified for full-blown diabetes.

"Type 2 diabetes" is defined as the form of diabetes that develops when the body does not respond properly to insulin, as opposed to type 1 diabetes, in which the pancreas makes no insulin at all. Type 2 diabetes mellitus results from a defect in insulin secretion and an impairment of insulin action in hepatic and peripheral tissues, especially muscle tissue and adipocytes. At first, the pancreas keeps up with the added demand by producing more insulin. In time, however, it loses the ability to secrete enough insulin in response to meals. A postreceptor defect also is present, causing resistance to the stimulatory effect of insulin on glucose use to occur, and relative insulin deficiency develops, unlike the absolute deficiency found in patients with type 1 diabetes. The specific etiologic factors are not known, but genetic input is much stronger in type 2 than in type 1.

"Metabolic syndrome" is described as a collection of health risks that increases the chance of developing heart disease, stroke, and diabetes. The syndrome is closely associated with the generalized metabolic disorder called insulin resistance. This is why metabolic syndrome is also called "insulin resistance syndrome", and it is also known by other names like "syndrome X" and "dysmetabolic syndrome". The risk factors include: obesity, insulin resistance or impaired glucose tolerance, hyperinsulinemia (high blood insulin levels), hypertension (high blood pressure), dyslipidemia (abnormal levels of triglycerides and HDL-cholesterol in blood) and proinflammatory state (e.g. elevated high-sensitivity C-reactive protein in blood).

"Obesity" is a condition characterized by a weight greater than what is generally considered healthy for a given height. Obesity can cause many health problems due to the strain it puts on organs and joints. It increases the risk of some widespread and potentially fatal disorders such as diabetes, high blood pressure, dyslipidemia (e.g., high total cholesterol or high levels of triglycerides), osteoarthritis, sleep apnea and respiratory problems, some cancers (endometrial, breast, and colon), coronary artery disease and stroke. It may also lead to psychological problems such as depression. Results of the National Health and Nutrition Examination Survey for 1999―2002 indicate that an estimated 30% of U.S. adults aged 20 years and older - over 60 million people - are obese, defined as having a body mass index (BMI) of 30 or higher (a BMI between 25 and 29.9 is considered overweight). An estimated 16% of children and adolescents ages 6-19 years are overweight and recent data in children as young as 2 years old throughout adolescence have indicated a 3-fold increase in the prevalence of overweight.

In a preferred embodiment the disease, as described herein, is a disease selected from the group consisting of: type 2 diabetes mellitus, obesity and metabolic syndrome.

### Human with high level of LBP / low level of BPI

As it can be seen in the working examples herein, the inventors have found that there is a negative correlation between LBP plasma levels and insulin sensitivity in subjects with glucose intolerance. In addition, LBP is negatively associated with glucose effectiveness. Furthermore, plasma LBP is positively associated with several components of the metabolic syndrome such as BMI and systolic blood pressure, fasting and post-load glucose concentrations, fasting insulin, glycated hemoglobin and fasting triglycerides.

Accordingly, a preferred embodiment of the invention relates to the use of a BPI protein product for the manufacture of a medicament for therapeutic or preventive treatment of a disease according to the first aspect of the invention, wherein the human is a human having a LBP plasma level higher than the median of healthy humans, which is 27 micrograms/ml. Preferably, the human has a LBP level higher than 35 micrograms/ml, and more particularly, higher than 45 micrograms/ml.

The art describes suitable methods for determining plasma LBP concentration in a human. It is preferred to use an ELISA based technique using antibodies recognizing human LBP as is described in the working examples (see EXAMPLE 1, section "LBP ELISA"). Levels 27, 35 and 45 micrograms/ml indicated above are according to this ELISA technique.

Since BPI and LBP are functionally antagonistic, instead of or in addition to test LBP plasma levels in a subject susceptible to be treated with a medicament comprising a BPI protein product, it could be useful to test BPI plasma levels as well. Accordingly, a human having a BPI plasma levels lower than 10 ng/ml and preferably lower than 7 ng/ml, will be susceptible to be treated with a medicament comprising a BPI protein product.

A suitable ELISA method using antibodies recognizing human BPI to determine plasma BPI concentration is in the working examples (see EXAMPLE 1, section "BPI ELISA"). Levels 10 and 7 ng/ml indicated above are according to this ELISA technique.

### Human with a G-to-A transition at 3' untranslated region of the human BPI protein gene

As it can be seen in the working examples herein, the inventors have found that the presence of an adenine instead of a guanine at 3' untranslated region of the human BPI protein gene (polymorphism identified as rs1131847 in NCBI, Entrez SNP) is associated with differences in plasma BPI concentration. This polymorphism is located at nucleotide 1524 (5'-3') of the complete BPI protein gene (NCBI NM_001725, Entrez Nucleotide). This nucleotide is the first one of the 3' untranslated region of the this gene, region identified as SH GC-12397. The inventors have found that A/- in this position correlates with low BPI and low insulin sensitivity, making the subject more susceptible to be treated with a medicament comprising a BPI protein product.

Accordingly, a preferred embodiment of the invention relates to the use of a BPI protein product for the manufacture of a medicament for therapeutic or preventive treatment of a disease indicated above in the first aspect of the invention, wherein the human is a human who carries an adenine in the polymorphism at 3' untranslated region of the human BPI protein gene, said polymorphism identified as rs1131847 in NCBI, Entrez SNP, and said polymorphism located at nucleotide 1524, 5'-3', of the complete BPI protein gene referenced as NCBI NM_001725, Entrez Nucleotide.

The skilled in the art will choose a suitable method to genotype if the subject susceptible to be treated with a medicament comprising a BPI protein product carries de A/- for this polymorphism (see below for further details).

### Administration of BPI

According to the teaching of the present invention BPI protein can be administrated to a mammalian (preferably a human) for therapeutic or preventive treatment of a disease involving insulin resistance. The purpose of the administration of BPI protein may be preventive (to avoid the development of these diseases) and/or therapeutic (to treat these diseases once they have been developed/installed). Preferably, BPI protein is administrated to a human. If administrated to an animal is it preferred that the animal is a mouse.

Suitable dosages for systemic or local administration include doses ranging from 1 µg/kg of body weight to 100 mg/kg per day or doses ranging from 0.1 mg/kg to 20 mg/kg per day. The amount may be administered, e.g., in divided doses on daily basis. The treatment may be continuous or by intermittent administration, at the same, reduced or increased dose per day for as long as determined by the treating, depending on the particular application or severity of the disease. Those who are skilled in the art may ascertain the proper dose using standard procedures. It is understood that the dose should be an effective amount of BPI protein in the sense that improved insulin resistance is seen in the treated subject. Suitable assay for testing improved insulin resistance is known to the skilled person and guidance may be found in the working examples herein.

According to common general knowledge it is understood that the medicament may be in the form of a pharmaceutical composition. Such medicament/pharmaceutical composition may be administered by any means that achieve their intended purpose. For example, administration may be by parenteral routes, including subcutaneous, intravenous, intradermal, intramuscular, intraperitoneal, intrathecal, transdermal, or buccal routes. Alternatively, or concurrently, administration may be by oral or rectal route. The pharmaceutical compositions can be administered parenterally by bolus injection or by gradual release over time.

As explained above, since BPI merely normalizes the glucose level, the risk of too low glucose levels is absent. This offers the possibility of e.g. administrating BPI protein as a depot (e.g. an injectable depot composition) to the patient. The depot can be made with an adequate release profile of BPI protein and the patient can thereby, in a comfortable easy way, get a continued improved insulin resistance and thereby continued normalized blood glucose levels.

Accordingly, in a preferred embodiment the medicament comprises a depot composition, more preferably an injectable depot composition. After introduction into the patient (animal or human), the depot shall preferably have an adequate release profile of BPI protein. Numerous suitable depot compositions are know to the skilled person and can be made with various adequate release profiles. See e.g. US 6331311 with title "Injectable depot gel composition and method of preparing the composition" for further details with respect to suitable depot compositions. Preferably, the injectable depot composition is an injectable depot gel composition.

Preferably, the depot composition is administrated by implanting, preferably subcutaneously, a suitable device. An example of such a suitable device is so-called pump. A suitable example of this is the commercial available ALZET^{®} Osmotic Pump from the company Durect Corp., USA. Suitable adequate pumps are known to the skilled person and they provide the possibility of providing continuous delivery (preferably by subcutaneously implantation), thereby eliminating the need for frequent, round-the-clock injections. Adequate release profiles could e.g. be a release profile allowing the depot to be administrated in a period interval from between each 14 days to each 3 months.

In addition to BPI protein, the pharmaceutical compositions may contain suitable pharmaceutically acceptable carriers comprising excipients and auxiliaries which facilitate processing of the active compounds into preparations which can be used pharmaceutically. Preferably, the preparations, particularly those which can be administered orally and which can be used for the preferred type of administration, such as tablets, dragees, and capsules, and also preparations which can be administered rectally, such as suppositories, as well as suitable solutions for administration by injection or orally, contain from about 0.1 to about 99 percent, preferably from about 25-85 percent, of active compound(s), together with the excipient.

Suitable excipients are, in particular, fillers such as sugars, such as lactose, sucrose, manitol, or sorbitol; cellulose preparations and/or calcium phosphates, such as tricalcium phosphate or calcium hydrogen phosphate; as well as binders such as starch paste made using, for example, maize starch, wheat starch, rice starch, potato starch, gelatin, gum tragacanth, methyl cellulose, hydroxypropylmethylcellulose, sodium carboxymethyl cellulose, and/or polyvinylpyrrolidone. If desired, disintegrating agents may also be added, such as the above-mentioned starches as well as carboxymethyl starch, cross-linked polyvinyl pyrrolidone, agar, or alginic acid or a salt thereof, such as sodium alginate. Auxiliaries which can be used in the compositions according to the present invention include flow-regulating agents and lubricants such as silica, talc, stearic acid or salts thereof, a detergent such as Triton, and/or polyethylene glycol.

A stable pharmaceutical composition containing BPI protein products (e. g., rBPI23) comprises the BPI protein product at a concentration of 1 mg/ml in citrate buffered saline (5 or 20 mM citrate, 150 mM NaCl, pH 5.0) comprising 0.1% by weight of poloxamer 188 (Pluronic F-68, BASF Wyandotte, Parsippany, NJ) and 0.002% by weight of polysorbate 80 (Tween 80, ICI Americas Inc., Wilmington, DE). Another stable pharmaceutical composition containing BPI protein products (e. g., rBPI21) comprises the BPI protein product at a concentration of 2 mg/ml in 5 mM citrate, 150 mM NaCl, 0.2% poloxamer 188 and 0.002% polysorbate 80. As described in WO 96/21436, other poloxamer formulations of BPI protein products with enhanced activity may be utilized, optionally with EDTA.

A preparation of the recombinant modified N-terminal fragment of human BPI (rBPI21 described above) undergoing trial is Neuprex^{®} (Xoma Corp).

It is contemplated that the administration of a BPI protein product may be accompanied by the concurrent administration of other therapeutic agents depending on the condition.

### Suitable kits

The kit according to the invention may advantageously be used to detect a predisposition to the above-mentioned diseases. The presence of the A/- allele is associated to an increased predisposition to have or develop said group of diseases.

The term predisposition, as used herein, is to be understood broadly, as the quality or state of being susceptible, the state of being predisposed to, sensitive to, or of lacking the ability to resist something (as a pathogen, familial disease, etc), or having an increased risk of developing a disease.

Appropriate means for the kit of the invention are those based on genotyping techniques well-known for a skilled in the art. These techniques should be able to read completely or partially BPI genotype or to distinguish selectively the polymorphism rs1131847 (NCBI, Entrez SNP) from other possible mutations on BPI gene. The presence of the polymorphism can be detected using one or more oligonucleotides which hybridize to a nucleotide sequence comprising the polymorphism on the BPI gene. Oligonucleotides can be fluorescently, chemiluminescently or radioactively labelled to act as probes and detect the nucleotide sequence comprising the polymorphism. These probes can be used for example in microarrays on glass support or in bead-based microarrays. Preferably, the means of the kit are based on PCR technology. Examples of PCR-based methods are restriction fragment length polymorphism (RFLP), site-directed mutagenesis (SDM), sequence-specific oligonucleotide (SSO) hybridization, nested primer, DNA heteroduplexes, or amplification refractory mutation system-PCR (ARMS-PCR).

An example of a suitable method to detect the polymorphism is in the working examples below (see EXAMPLE 2, section "Methods").

In a particular embodiment of the invention, the kit further comprises appropriate instructions for carrying out the detection. Instructions may comprise those rules on how to make use of the reagents and instrumentation suitable to carry out the detection of the presence of the polymorphism on the BPI gene. Furthermore, instructions may also comprise those rules on how to interpret the results and link the results with the predisposition to suffer from the group of diseases described in this invention. An example of this can be defined by fluorescence patterns for comparing the detected signals in real time PCR.

Accordingly, in a preferred embodiment, the kit further comprises appropriate instructions explaining that the presence of the A/- allele for this position is associated to an increased predisposition to have or develop said group of diseases.

The detection is performed with a separated sample from the human susceptible to suffer from the disease. The sample is a tissue or a fluid, generally blood, taken from the individual. Depending on the selected technique, the sample will be processed to obtain isolated cells, protein fraction or nucleic acids fraction (e.g. genomic DNA or messenger RNA).

### EXAMPLES:

The general aim of the following examples was to study whether the production of BPI could be linked to insulin sensitivity and glucose tolerance, and to study whether the capacity of BPI production could lead to differences in insulin sensitivity.

### EXAMPLE 1: Study of circulating BPI across categories of glucose tolerance

The objective of this study was to evaluate circulating BPI in healthy subjects, in patients with glucose intolerance and in patients with type 2 diabetes mellitus.

### Subjects and methods

One hundred and seventy-four Caucasian subjects were recruited and studied, including the analysis of glucose tolerance and insulin sensitivity, within an ongoing studied dealing on non-classical cardiovascular risk factors. All normotolerant subjects (n=114) had fasting plasma glucose < 7.0 mM and two-hour post-load plasma glucose < 11.1 mM after a 75 g oral glucose tolerance test. Glucose intolerance was diagnosed in 60 subjects according to the American Diabetes Association Criteria (post-load glucose between 7.8 and 11.1 mmol/l). Inclusion criteria were 1) BMI < 40 kg/m², 2) absence of systemic disease, and 3) absence of infection within the previous month. None of the control subjects were under medication or had evidence of metabolic disease other than obesity. Alcohol and caffeine were withheld within 12 h of performing the insulin sensitivity test. Smokers were defined as any person consuming at least 1 cigarette a day in the previous 6 months. Resting blood pressure was measured as previously reported (Fernández-Real JM, et al., J Clin Endocrinol Metab 2003, vol. 88, pp. 1780-4). Liver disease and thyroid dysfunction were specifically excluded by biochemical work-up.

Patients with type 2 diabetes, according to American Diabetes Association criteria, were prospectively recruited from diabetes outpatient clinics on the basis of a stable metabolic control in the previous 6 months, as defined by stable HbA1c values. Exclusion criteria included the following: 1) clinically significant hepatic, neurological, endocrinologic, or other major systemic disease, including malignancy; 2) history or current clinical evidence of hemochromatosis; 3) history of drug or alcohol abuse, defined as >80 g/day in men and >40 g/day in women, or serum transaminase activity more than twice the upper limit of normal; 4) an elevated serum creatinine concentration; 5) acute major cardiovascular event in the previous 6 months; 6) acute illnesses and current evidence of acute or chronic inflammatory or infective diseases; and 7) mental illness rendering the subjects unable to understand the nature, scope, and possible consequences of the study. Pharmacological treatment for these patients was: Insulin: 44.8%; oral hypoglycaemic agents: 72.9%; statins: 38.0%; fibrates: 10.6%; blood pressure lowering agents: 61.5%; aspirin: 42.7%; and allopurinol: 4.2%. All subjects gave written informed consent after the purpose of the study was explained to them. The institutional review board of the institution approved the protocol.

Anthropometric and clinical measurements: Body mass index (BMI) and waist-to-hip ratio (WHR) were measured as previously reported (Fernández-Real JM, et al., J Clin Endocrinol Metab 2003, vol. 88, pp. 1780-4). Fat mass (FM) and percent fat mass (PFM) were calculated using bioelectric impedance (Holtain BC Analyzer, UK).

Study of insulin sensitivity: Insulin sensitivity and glucose effectiveness were measured in a sample of subjects using the frequently sampled intravenous glucose tolerance test on a different day, as previously described (Fernández-Real JM, et al., J Clin Endocrinol Metab 2003, vol. 88, pp. 1780-4).

Analytical methods: Serum glucose concentrations were measured in duplicate by the glucose oxidase method using a Beckman glucose analyzer II (Beckman Instruments, Brea, California). In the clamp studies, plasma glucose was measured immediately using a glucose analyzer YSI 2300 STAT Plus (YSI Incorporated, Yellow Springs, Ohio). Total serum cholesterol was measured through the reaction of cholesterol esterase/cholesterol oxidase/peroxidase. Total serum triglycerides were measured through the reaction of glycerol-phosphate-oxidase and peroxidase. Uric acid was measured by routine laboratory tests. HbA1c was measured by the high-performance liquid chromatography method (Bio-Rad, Muenchen, Germany, and autoanalyzer Jokoh HS-10, respectively). Intraassay and interassay coefficients of variation were less than 4% for all these tests. Serum insulin levels were measured in duplicate by monoclonal immunoradiometric assay (IRMA or enzyme amplified sensitivity immunoassay (EASIA), Medgenix Diagnostics, Fleunes, Belgium). Intraassay and interassay coefficients of variation were similar to those previously reported (Fernández-Real JM, et al., J Clin Endocrinol Metab 2003, vol. 88, pp. 1780-4).

BPI ELISA: Plasma EDTA BPI concentrations were measured by a sandwich enzyme-linked immunosorbent assay (Human BPI ELISA kit, HyCult biotechnology b.v.; PB Uden, The Netherlands) according to the manufacturer's instructions. The kit has an assay sensitivity of 250 pg/ml. Intra- and interassay coefficients of variation were <5%.

LBP ELISA: Plasma and serum LBP levels were determined with a commercially available Human LBP ELISA kit (HyCult biotechnology b.v.; PB Uden, The Netherlands). Plasma and serum samples were diluted at least 1000 times and assayed according to the manufacturer's instructions. The kit has an assay sensitivity of 1 ng/ml and a measurable concentration range of 0.8 to 50 ng/ml. Intra- and interassay coefficients of variation were between 5-10%.

Statistical methods: Descriptive results of continuous variables are expressed as mean ± SD. Parameters that did not follow normal distribution (SI, SG, triglycerides, LBP, BPI) were log transformed. Values were then expressed as the antilog transformed variables of the mean log transformed values. Differences between groups were assessed by Student's t test, Mann-Whitney test, as appropriate. For the effects of treatment, Wilcoxon rank's test was used. The relationships between quantitative variables were analyzed by simple correlation using log-transformed variables (Pearson's test) and by stepwise multivariate linear regression analysis. Independent variables were considered. Levels of statistical significance were set at p < 0.05.

### Results

**Table 1: Characteristics of the subjects (anthropometrical and biochemical variables)**

| | Normotolerant men | Glucose-intolerant men | P | Type-2 diabetic patients |
|---|---|---|---|---|
| N | 114 | 60 | - | 170 |
| Age (years) | 46.2 ± 11.9 | 53.2 ± 11.2^{‡} | <0.0001 | 57.2 ± 11.8 |
| Weight (kg) | 78 ± 12.1 | 84.4 ± 12^{†} | 0.002 | 85.5 ± 19 |
| Body mass index (kg/m²) | 27.01 ± 3.6 | 29.5 ± 3.9^{‡} | <0.0001 | 32.3 ± 7.0 |
| Systolic blood pressure (mm Hg) | 124 ± 14.5 | 132.8 ± 15.9^{‡} | 0.004 | 140.0 ± 21.5 |
| Diastolic blood pressure (mm Hg) | 78.3 ± 10.7 | 83.4 ± 9.8^{†} | 0.8 | 81.2 ± 10.8 |
| Waist-to-hip ratio | 0.92 ± 0.06 | 0.95 ± 0.06^{†} | 0.009 | - |
| Fat-free mass (kg) | 72.1 ± 9.9 | 71.8 ± 9.2 | <0.0001 | - |
| Fat-mass* (kg) | 6.1 (2.6 - 13.2) | 10.22 (5.24 - 18.27)^{†} | 0.002 | - |
| **Fasting insulin (mU/l)** | **8.23 ± 4.1** | **11.8 ± 5.7^{‡}** | **<0.0001** | - |
| **Fasting glucose (mmol/l)** | **5.15 ± 0.4** | **5.9 ± 1.0^{‡}** | **<0.0001** | **10.0 ± 4.7** |
| HbA_{1c} (%) | 4.78 ± 0.34 | 5.00 ± 0.58^{†} | 0.003 | 7.3 ± 1.7 |
| HDL-Cholesterol (mg/dl) | 52.8 ± 12.1 | 51.1 ± 10.7 | 0.3 | 50.5 ± 16.4 |
| Triglycerides (mg/dl) | 97 ± 54.6 | 121.3 ± 63.2* | 0.015 | 223.6 ± 142.0 |
| Insulin sensitivity (10⁻⁴* mU/L)* | 2.72 (1.95 - 4.39) | 1.32 (0.77 - 2.24)^{‡} | <0.0001 | - |
| Glucose effectiveness* | 0.020 (0.016- 0.024) | 0.018 (0.014-0.020)* | 0.01 | - |
| **BPI (ng/ml)*** | **14.22** **(6.6 - 28.5)** | **17.66** **(8.6 - 31)** | **0.51** | **10.64** **(6.0 -19.1)** |
| LBP (µg/ml)* | **27.82** **(9.8 - 50.1)** | **17.26** **(9.59 - 44.93)** | **0.47** | |

| | | | | |
|---|---|---|---|---|
| * <0.05; ^{†}<0.01; and^{‡} <0.0001, compared to normotolerant men; ** <0.05; ^{††} <0.01; and ^{‡‡} <0.0001, compared to glucose-intolerant men | | | | |

Conclusions from Table 1: BPI was significantly higher among patients with glucose intolerance compared to control subjects and patients with type 2 diabetes. Circulating BPI correlated negatively with LBP (t=-0.31, p<0.0001). This relationship was especially significant among normotolerant subjects (r=-0.35, p<0.0001) but not in subjects with glucose intolerance (r=-0.18, p=0.16). Circulating BPI results were parallel to results for fasting insulin. A possible explanation behind this fact is that in subjects with glucose intolerance, insulin goes up since insulin sensitivity is decreased. BPI also goes up to help insulin to reestablish the levels of glucose but sometimes BPI supply is not enough. This leads to a lower insulin sensitivity and to the onset of type 2 diabetes. If BPI is given to glucose intolerants, it could help to improve insulin resistance and to prevent the development of type 2 diabetes.

**Table 2. Correlationships between circulating BPI, LBP and clinical variables.**

| | | All subjects (n=174) | P | Normotolerant subjects (n=114) | P | Glucose-intolerant subjects (n=60) | P |
|---|---|---|---|---|---|---|---|
| Age | BPI | **0.19** | **0.02** | 0.14 | NS | **0.30** | **0.02** |
| | LBP | **-0.22** | **0.007** | **-0.29** | **0.005** | -0.12 | NS |
| BMI | BPI | -0.13 | 0.07 | -0.07 | NS | **-0.26** | **0.04** |
| | LBP | **0.21** | **0.005** | 0.13 | 0.1 | **0.40** | **0.002** |
| WHR | BPI | -0.12 | 0.1 | 0.04 | NS | **-0.39** | **0.002** |
| | LBP | 0.12 | 0.1 | 0.04 | NS | 0.20 | 0.1 |
| Fat-mass | BPI | -0.12 | 0.1 | 0.04 | NS | -0.24 | 0.08 |
| | LBP | -0.10 | NS | -0.19 | NS | 0.04 | NS |
| Fat-free mass | BPI | 0.03 | NS | 0.006 | NS | 0.04 | NS |
| | LBP | **0.19** | **0.04** | 0.17 | 0.1 | 0.17 | NS |
| SBP (mm Hg) | BPI | 0.04 | NS | 0.05 | NS | -0.03 | NS |
| | LBP | 0.02 | NS | 0.008 | NS | 0.15 | NS |
| DBP (mm Hg) | BPI | 0.08 | NS | 0.10 | NS | 0.005 | NS |
| | LBP | -0.06 | NS | -0.10 | NS | **0.30** | **0.02** |

| | | | | | | | |
|---|---|---|---|---|---|---|---|
| (BMI, body mass index; WHR, waist-to-hip ratio; SBP, systolic blood pressure; DBP, diastolic blood pressure) | | | | | | | |

Conclusions from Table 2: The strongest associations between plasma BPI and central obesity, glucose metabolism, insulin sensitivity and components of the metabolic syndrome were found in subjects with glucose intolerance. In this subjects, BPI levels correlate negatively with both body mass index and waist-to-hip ratio.

**Table 3. Correlationships between circulating BPI, LBP and biochemical variables.**

| | | All subjects (n=174) | P | Normotolerant subjects (n=114) | P | Glucose-intolerant subjects (n=60) | P |
|---|---|---|---|---|---|---|---|
| Fasting glucose | BPI | **-0.28** | **0.001** | -0.05 | NS | **-0.47** | **<0.0001** |
| | LBP | 0.14 | 0.09 | -0.01 | NS | **0.37** | **0.006** |
| 30' OGTT | BPI | -0.05 | NS | 0.06 | NS | -0.10 | NS |
| | LBP | -0.02 | NS | 0.03 | NS | 0.30 | 0.03 |
| 60' OGTT | BPI | -0.10 | NS | 0.05 | NS | -0.23 | 0.07 |
| | LBP | -0.02 | NS | 0.07 | NS | 0.43 | 0.001 |
| 90' OGTT | BPI | -0.10 | NS | 0.09 | NS | -0.25 | 0.05 |
| | LBP | 0.09 | NS | -0.11 | NS | **0.47** | **<0.0001** |
| 120' OGTT | BPI | -0.20 | 0.02 | -0.16 | 0.1 | **-0.29** | **0.02** |
| | LBP | 0.21 | 0.018 | -0.08 | NS | **0.47** | **<0.0001** |
| HbA_{1c} | BPI | **-0.19** | **0.03** | 0.11 | NS | **-0.39** | **0.009** |
| | LBP | **0.17** | **0.05** | -0.12 | NS | 0.35 | 0.002 |
| Fasting insulin | BPI | **-0.24** | **0.003** | -0.04 | NS | -0.41 | 0.001 |
| | LBP | 0.10 | NS | -0.03 | NS | 0.30 | 0.02 |
| 120' OGTT insulin | BPI | -0.17 | **0.04** | -0.13 | NS | -0.15 | NS |
| | LBP | 0.11 | 0.1 | 0.03 | NS | 0.24 | 0.08 |
| Triglycerides | BPI | -0.03 | NS | 0.08 | NS | -0.32 | 0.01 |
| | LBP | 0.11 | 0.1 | 0.04 | NS | 0.31 | 0.02 |
| HDL-cholesterol | BPI | **0.16** | **0.03** | **0.19** | **0.03** | 0.10 | NS |
| | LBP | -0.05 | NS | -0.007 | NS | -0.21 | 0.1 |
| C-reactive protein | BPI | 0.01 | NS | -0.03 | NS | 0.05 | NS |
| | LBP | **0.26** | **0.001** | **0.27** | **0.004** | **0.33** | **0.01** |
| sTNFR1 | BPI | -0.09 | NS | -0.14 | NS | -0.01 | NS |
| | LBP | 0.10 | NS | 0.002 | NS | **0.32** | **0.02** |
| sTNFR2 | BPI | **0.34** | **<0.001** | **0.36** | **<0.0001** | 0.20 | NS |
| | LBP | -0.16 | 0.07 | **-0.23** | **0.02** | 0.03 | NS |
| S_{I} | BPI | 0.07 | NS | 0.02 | NS | **0.31** | **0.015** |
| | LBP | -0.02 | NS | 0.05 | NS | **-0.40** | **0.002** |
| S_{G} | BPI | 0.05 | NS | 0.06 | NS | -0.06 | NS |
| | LBP | **-0.19** | **0.015** | -0.16 | 0.07 | -0.23 | 0.07 |
| WBC count | BPI | **0.21** | **0.01** | 0.17 | 0.1 | **0.28** | **0.02** |
| | LBP | 0.12 | NS | 0.07 | NS | 0.20 | NS |
| Neutrophil count | BPI | **0.25** | **0.002** | **0.24** | **0.02** | **0.30** | **0.02** |
| | LBP | 0.06 | NS | 0.02 | NS | 0.11 | NS |
| Eosinophil count | BPI | -0.08 | NS | -0.05 | NS | -0.13 | NS |
| | LBP | 0.17 | 0.04 | 0.07 | NS | **0.34** | **0.01** |

| | | | | | | | |
|---|---|---|---|---|---|---|---|
| (OGTT, oral glucose tolerance test; 30', 60', 90' and 120' indicate the minutes after glucose intake; sTNFR1, sTNFR2: soluble fraction of tumor necrosis factor α receptor 1 and 2, respectively; S_{I}, insulin sensitivity; S_{G}, glucose effectiveness) | | | | | | | |

Conclusions from Table 3: LBP was positively associated with fat-free mass (see Table 2) and C-reactive protein. Plasma LBP was positively associated with several components of the metabolic syndrome such as BMI and blood pressure (see Table 2), fasting and post-load glucose concentrations, fasting insulin, glycated hemoglobin and fasting triglycerides and negatively with insulin sensitivity in subjects with glucose intolerance. In addition, LBP was negatively associated with glucose effectiveness when all subjects were considered as a whole. Thus, in normotolerant subjects, there is no correlation between levels of BPI/LBP and glucose metabolism (NS results) whereas in glucose-intolerant subjects these levels are important for glucose levels.

### EXAMPLE 2: Study of the effects of a 3' UTR BPI gene polymorphism on circulating BPI and insulin action among nondiabetic subjects

The objective of this study was to evaluate whether a 3' UTR BPI polymorphism led to differences in BPI and insulin action among nondiabetic subjects.

### Methods

Genotyping: Genomic DNA was extracted from peripheral blood leukocytes according to standard procedures (QIAamp^{®} DNA Blood Mini Kit, Qiagen, Germany). For the detection of the polymorphism rs1131847 (NCBI, Entrez SNP), G-to-A transition, at 3' untranslated region (3'UTR) a method based on the TaqMan technology suitable for allelic discrimination (ABI Prism 7000 Sequence Detection System, Applied Biosystems, Darmstadt, Germany) was used. The samples were genotyped using an Applied Biosystems Taqman assay (assay-on-demand C_308491_1_) using a minor-groove binding reporter probes (VIC_labelled for the A allele and FAM-labelled for the G), and an end-read protocol. The PCR conditions were as recommended by the manufacturer and a sample containing water instead of DNA, as a negative control, were used for each PCR run.

### Results

**Table 4. BPI 3'UTR polymorphism and insulin sensitivity.**

| | A/- subjects | GG homozygotes | P |
|---|---|---|---|
| Men (n) | 115 | 59 | - |
| Normotolerant/glucose intolerant | 73/42 | 42/17 | 0.45 |
| Age (years) | 49.9 ±12.3 | 47.0 ± 12.2 | 0.14 |
| Weight (kg) | 80.8 ± 13.1 | 79.1 ± 10.4 | 0.42 |
| Body mass index (kg/m²) | 28.12 ± 4.1 | 27.5 ± 3.4 | 0.33 |
| **Waist-to-hip ratio** | **0.94 ± 0.07** | **0.91 ± 0.07** | **0.03** |
| Fat-free mass (kg) | 71.6 ± 9.8 | 71.7 ± 8.9 | 0.98 |
| Fat-mass* (kg) | 7.0 (2.6 - 15.1) | 8.5 (3.97 - 14.66) | 0.69 |
| Systolic blood pressure (mm Hg) | 127 ± 15.6 | 125.5 ± 14.2 | 0.44 |
| Diastolic blood pressure (mm Hg) | 79.8 ± 10.7 | 79.8 ± 10.1 | 0.99 |
| Fasting glucose (mmol/l) | 5.43 ± 0.76 | 5.46 ± 0.63 | 0.82 |
| Post-load glucose 120' (mmol/l) | 7.52 ± 2.60 | 6.88 ± 2.20 | 0.12 |
| **Fasting insulin (mU/l)** | **10.73 ± 5.7** | **8.26 ± 3.6** | **0.002** |
| **Post-load insulin 30' (mU/l)** | **79.9 ± 50.2** | **58.7 ± 32.7** | **0.002** |
| **Post-load insulin 120' (mU/l)*** | **57 (30.4 - 92.7)** | **41.2 (24.6 - 73.3)** | **0.04** |
| HbA_{1c} (%) | 4.86 ± 0.47 | 4.73 ± 0.44 | 0.07 |
| HDL-Cholesterol (mg/dl) | 51.6 ± 10.9 | 53.0 ± 13.1 | 0.47 |
| **Triglycerides (mg/dl)** | **116.8 ± 75.2** | **94.1 ± 54.2** | **0.026** |
| **Insulin sensitivity (10⁻⁴*mU/L)*** | **1.98 (1.1 - 3.05)** | **2.56 (1.74 - 4.29)** | **0.008** |
| Glucose effectiveness* | 0.019 (0.015- 0.024) | 0.019 (0.016 - 0.023) | 0.79 |
| **BPI (ng/ml)*** | **17.4 (7.8 - 30.7)** | **23.85 (10.1 - 44.4)** | **0.01** |
| **LBP (µg/ml)*** | 20.23 (8.5 - 43.3) | 14.04 (9.65 - 41.3) | 0.88 |

| | | | |
|---|---|---|---|
| (*expressed as median and interquartile range) | | | |

Conclusions from Table 4: This polymorphism led to differences in plasma BPI concentration. A/- correlates with lower BPI and lower insulin sensitivity. Interestingly, GG homozygotes showed significantly lower waist-to-hip ratio, lower fasting and post-load insulin concentration, lower plasma triglycerides and higher insulin sensitivity index than A/- subjects.

### EXAMPLE 3: Study of the effects of an insulin sensitizer (metformin) on BPI

The objective of this study was to evaluate the effects of changing insulin action -after treatment with an insulin sensitizer (metformin)- on circulating BPI in subjects with glucose intolerance.

### Methods

Patients were recruited from the Diabetes and Endocrinology Unit at Hospital de Girona Dr Josep Trueta. They were offered participation in the study if they were between 30 and 65 years old and fulfilled all the inclusion criteria. Men and women were both included, but women only when surgical sterility was documented or when they were post-menopausal or when a reliable method of contraception was used. The inclusion criteria were as follows: a) body mass index (BMI) between 22 and 35 kg/m², b) impaired glucose tolerance by an OGTT performed two months before the beginning of the study or a fasting glucose level between 110 and 140 mg/dl, c) stability of diet and physical exercise within the past two months. All subjects signed a written informed consent. Exclusion criteria were: a) diabetic patients according to NDDG criteria of 1979, b) pregnant and nursing women, c) patients with renal impairment defined as plasma creatinine values at or above 1.5 mg/dl for men and 1.4 mg/dl for women, d) patients affected by cardiac or respiratory insufficiency likely to cause central hypoxia or reduced peripheral perfusion, e) past history of lactic acidosis, f) non controlled hypertension, g) acute or chronic infection, h) liver disease, including alcoholic liver disease as demonstrated by abnormal liver-function tests or alcohol abuse, i) patients taking drugs that could modify glucose tolerance, j) participants in another clinical trial within the last 30 days, and k) legal incapacity as a study patient.

The clinical study was performed in accordance with the Declaration of Helsinki (revised version of Hong Kong 1989) as well as with the EC Note for guidance "Good clinical practice for studies on medicinal products in the European Community". It was approved by the local Ethics Committee and the Spanish Health Department (Clinical assay number: 97/337).
Thirty-one patients were randomized either to placebo or metformin according to the randomization tablets supply generated by LIPHA using the computer program RANCODE +3.1. A dietician with the aim of assuring a stable weight gave general diabetic dietary advice at the beginning of the study. Two months after the first visit (visit 2) subjects started taking metformin or placebo, one tablet per day (850 mg) for the first week and then two tablets per day (one after breakfast and one after dinner) for the following eleven weeks. Drug compliance was checked by tablet counts and any side effect was recorded at visit 3 (6 weeks after randomization) and at visit 4 (at the end of the study).

To assess glucose tolerance, an OGTT was performed after an overnight fast. Insulin sensitivity was evaluated by HOMA (Homeostasis model assessment) using basal (mean of three samples obtained at 5 minutes interval) glucose and insulin (Bonora E, et al., Diabetes Care, 2000, vol. 23, pp. 57-63). Beta cell function was calculated by CIGMA (Continuous infusion of glucose with model assessment) from achieved C-peptide and glucose values (Hosker JP, et al., Diabetologia 1985, vol. 28, pp. 401-11). CIGMA test consists of a continuous intravenous infusion of 5-mg glucose per kg of ideal body weight.min⁻¹ using a 10 g.dl⁻¹glucose solution with model assessment of glucose, insulin and C-peptide (RIA, Byk-Sangtec Diagnostica, Dietzenbach, Germany) values obtained before (basal value, mean value of three samples obtained at 5 minute intervals) and at the end (achieved value, mean value of samples obtained at 50, 55 and 60 minutes) of the test. C-peptide detection level was 0.1 ng/ml and had intra- and inter-assay coefficients of variation of 2.6% and 4.4 %. It shows 25% cross-reactivity with proinsulin but not with insulin.

Out of 118 pre-selected subjects, 31 patients were randomized for the study (16 to metformin and 15 to placebo). Six patients (3 on metformin and 3 on placebo) discontinued early and two non-compliant patients were excluded (both in metformin group). 21 patients (9 on metformin and 12 on placebo) were finally analyzed.

### Results

**Table 5. Study of the effects of an insulin sensitizer (metformin) on circulating BPI.**

| | **Metformin** **Baseline** | **Metformin** **12 weeks** | **Placebo** **Baseline** | **Placebo** **12 weeks** |
|---|---|---|---|---|
| Sex (M/F) | 4/5 | - | 5/7 | - |
| Age (years) | 46.7 ± 7.8 | - | 46.5 ± 6.7 | - |
| BMI (Kg/m²) | 28.0 ± 4.5 | 27.7 ± 4.3 | 28.8 ± 4.0 | 28.4 ± 3.8 |
| Waist circumference | 95.9 ± 11.5 | 94.8 ± 9.4 | 95.5 ± 10.4 | 93.9 ± 10.6 |
| Waist-to-hip ratio | 0.96 ± 0.1 | 0.92 ± 0.07 | 0.91 ± 0.07 | 0.92 ± 0.07 |
| Fasting glucose (mg/dl) | 110.1 ± 9.9 | **98.9 ± 15.7^{a}** | 106.9 ± 8.9 | **106.3 ± 6.5** |
| 120' OGTT glucose (mg/dl) | 162.2 ± 27.8 | **140.6 ± 42.9** | 130.3 ± 31 | **139.5 ± 28.5** |
| Fasting insulin (mU/l) | 11.6 ± 5.4 | **8.8 ± 3.5^{b}** | 11.2 ± 3.3 | **11.5 ± 3.3** |
| Fasting C-peptide (ng/ml) | 2.5 ± 0.7 | 1.8 ± 0.5^{b} | 2.1 ± 0.7 | 2.0 ± 0.8 |
| Achieved glucose (mg/dl) | 190.5 ± 18.9 | 174.6 ± 34.5 | 194.8 ± 17.1 | 180.9 ± 13.9 |
| Achieved insulin (mU/l) | 28.5 ± 13.5 | 23.3 ± 7.6 | 27.7 ± 11.3 | 31.3 ± 14.2 |
| Achieved C-peptide (ng/ml) | 5.2 ± 1.2 | 4.2 ± 1.0^{b} | 4.4 ± 1.4 | 4.5 ± 1.5 |
| HOMA sensitivity (%) | 37.4 ± 15.2 | **50.4** ± **23.2^{b}** | 35.4 ± 10.1 | **34.6 ± 9.9** |
| CIGMA beta cell function (%) | 82.8 ± 24.0 | 90.9 ± 34.0 | 71.7 ± 27.2 | 83.1 ± 30.0 |
| BPI (ng/ml)* | 2.75 (1.26-7.73) | 7.49 (2.56-38.8)^{c} | 3.24 (1.58-5.3) | 2.34 (1.06-8.2) |

| | | | | |
|---|---|---|---|---|
| (Achieved glucose, insulin and C-peptide refers to these parameters after intravenous glucose. Data are means ± SD. ^{a}p<0.01, ^{b}p<0.05, ^{c}p=0.017 (within group), except for *expressed as median -interquartile range-) | | | | |

Conclusion from Table 5: Patients were of similar age, BMI and sex. Fasting glucose, insulin and C-peptide levels were similar. Insulin sensitivity and beta cell function were also nonsignificantly different. After 12-week treatment, fasting glucose decreased in the metformin group. Fasting insulin, fasting C-peptide and achieved C-peptide levels were also statistically lower following metformin treatment. Insulin sensitivity by HOMA improved after metformin treatment and was not modified by placebo. Beta cell function was not modified in either group.
In parallel to improved insulin sensitivity, plasma BPI significantly increased only in the metformin group. In conclusion, administering metformin, insulin sensitivity is improved and levels of BPI are higher.

## Claims

1. Use of a bactericidal/permeability-increasing (BPI) protein product for the manufacture of a medicament for therapeutic or preventive treatment of a disease involving a clinical disorder associated with insulin resistance in a human, in order to lower the level of glucose in blood and thereby get a normalization of the level of glucose, wherein the disease is a disease selected from the group consisting of: type 2 diabetes mellitus, obesity, glucose intolerance, metabolic syndrome , arterial hypertension, cardiovascular disease, dyslipidemia, and functional ovaric hyperandrogenism.

2. The use according to claim 1, wherein the disease is obesity.

3. The use according to claim 1, wherein the disease is type 2 diabetes mellitus.

4. The use according to claim 1, wherein the disease is metabolic syndrome.

5. The use according to any of the preeceding claims, wherein the human is a human having a lipopolysaccharide binding protein (LBP) plasma level higher than 27 micrograms/ml, level determined by a LBP ELISA assay using a human LBP ELISA kit with antibodies recognizing human LBP.

6. The use according to any of the preceding claims, wherein the human is a human who carries an adenine in the polymorphism at 3' untranslated region of the human BPI protein gene, said polymorphism identified as rs1131847 in NCBI, Entrez SNP, and said polymorphism located at nucleotide 1524, 5'-3', of the complete BPI protein gene referenced as NCBI NM_001725, Entrez Nucleotide.

7. The use according to any of the preceding claims, wherein the BPI protein product is a human BPI protein product that comprises the following amino acid sequence:
V N P G V V V R I S Q K G L D Y A S Q Q G T A A L Q K E L K R I K I P D Y S D S F K I K H L G K G H Y S F Y S M D I R E F Q L P S S Q I S M V P N V G L K F S I S N A N I K I S G K W K A Q K R F L K M S G N F D L S I E G M S I S A D L K L G S N P T S G K P T I T A S S C S S H I N S V H V H I S K S K V G W L I Q L F H K K I E S A L R N K M N S Q V C E K V T N S V S S K L Q P Y F Q T L

8. The use according to any of the preceding claims, wherein the medicament comprises an injectable depot composition, which after introduction into the human has an adequate release profile of BPI protein product.

9. The use according to claim 8, wherein the depot composition is administrated by implanting, preferably subcutaneously, a suitable device in the animal or human, preferably the suitable device is a pump.

10. A kit for detecting a predisposition to a disease involving a clinical disorder associated with insulin resistance in a human, wherein the disease is a disease according to claim 1, comprising appropriate means for detecting, in a separated sample from the human, the presence or the absence of an adenine in the polymorphism at 3' untranslated region of the human BPI protein gene, said polymorphism identified as rs1131847 in NCBI, Entrez SNP, and said polymorphism located at nucleotide 1524, 5'-3', of the complete BPI protein gene referenced as NCBI NM_001725, Entrez Nucleotide.


SEQUENCE LISTING

<110>  Mellitus, SL

<120>  Use of BPI protein for treatment of diseases

<130>  P593EP00

<160>  4

<170>  PatentIn version 3.1

<210>  1
<211>  193
<212>  PRT
<213>  Homo sapiens (modified Cys132Ala)

<400>  1

Val Asn Pro Gly Val Val Val Arg Ile Ser Gln Lys Gly Leu Asp Tyr
1               5                   10                  15

Ala Ser Gln Gln Gly Thr Ala Ala Leu Gln Lys Glu Leu Lys Arg Ile
            20                  25                  30

Lys Ile Pro Asp Tyr Ser Asp Ser Phe Lys Ile Lys His Leu Gly Lys
        35                  40                  45

Gly His Tyr Ser Phe Tyr Ser Met Asp Ile Arg Glu Phe Gln Leu Pro
    50                  55                  60

Ser Ser Gln Ile Ser Met Val Pro Asn Val Gly Leu Lys Phe Ser Ile
65                  70                  75                  80

Ser Asn Ala Asn Ile Lys Ile Ser Gly Lys Trp Lys Ala Gln Lys Arg
                85                  90                  95

Phe Leu Lys Met Ser Gly Asn Phe Asp Leu Ser Ile Glu Gly Met Ser
            100                 105                 110

Ile Ser Ala Asp Leu Lys Leu Gly Ser Asn Pro Thr Ser Gly Lys Pro
        115                 120                 125

Thr Ile Thr Ala Ser Ser Cys Ser Ser His Ile Asn Ser Val His Val
    130                 135                 140

His Ile Ser Lys Ser Lys Val Gly Trp Leu Ile Gln Leu Phe His Lys
145                 150                 155                 160

Lys Ile Glu Ser Ala Leu Arg Asn Lys Met Asn Ser Gln Val Cys Glu
                165                 170                 175

Lys Val Thr Asn Ser Val Ser Ser Lys Leu Gln Pro Tyr Phe Gln Thr
            180                 185                 190

Leu

<210> 2
<211> 29
<212> PRT
<213> Homo sapiens

<400> 2

Ala Ser Gln Gln Gly Thr Ala Ala Leu Gln Lys Glu Leu Lys Arg Ile
1               5                   10                  15

Lys Ile Pro Asp Tyr Ser Asp Ser Phe Lys Ile Lys His
            20                  25

<210> 3
<211> 35
<212> PRT
<213> Homo sapiens

<400> 3

Ser Ser Gln Ile Ser Met Val Pro Asn Val Gly Leu Lys Phe Ser Ile
1               5                   10                  15

Ser Asn Ala Asn Ile Lys Ile Ser Gly Lys Trp Lys Ala Gln Lys Arg
            20                  25                  30

Phe Leu Lys
        35

<210> 4
<211> 28
<212> PRT
<213> Homo sapiens

<400> 4

Val His Val His Ile Ser Lys Ser Lys Val Gly Trp Leu Ile Gln Leu
1               5                   10                  15

Phe His Lys Lys Ile Glu Ser Ala Leu Arg Asn Lys
            20                  25